# EUROPEAN PATENT APPLICATION

(11) **EP 0 887 043 A1**
(43) Date of publication of application: **30.12.1998**
(21) Application number: 98304796.0
(22) Date of filing: 17.06.1998
(51) Int. Cl.: A61B 1/04, A61B 1/00

(54) **Protective window for endoscope**

(30) Priority: 19.06.1997 US 50182 P; 11.06.1998 US 95596
(71) Applicant: Galileo Corporation, Sturbridge, MA 01566 (US)
(72) Inventor: Deary, Randall, Acton, MA 01720 (US); Shoemaker, Arthur H., Dudley, MA 01571 (US); Magnant, Richard, Southbridge, MA 01550 (US)
(74) Representative: Brunner, Michael John

(57) **Abstract**

The imaging or proximal end surface (18) of a fibre optic endoscope (10) may be coupled to a viewing optic (20). The proximal end surface (18) is protected by means of an optically clear window (30) which is disposed either in contact with the proximal end (18) of the fibre by means of an index matching adhesive (36) or is separated from such proximal end (18). The fibre is positionable with respect to a viewing optic (20) having a focal point (F) on the image plane (18) of the fibre (12). In the former case, both surfaces (32,34) of the window are out of focus. In the latter case only, the remote surface (34) is out of focus.

## Description

The invention is directed to a device for protecting the end of an imaging fibre. In particular, the invention is directed to an arrangement for positioning the imaging end of a fibre optic endoscope window and out of the focal plane of the optical fibre.

The size of an imaging fibre used in exemplary endoscopes discussed herein ranges from about 0.18mm in diameter to about 1.025mm in diameter. Using lens technology, images, sometimes referred to as aerial images, may be formed away from any optical surface. With an imaging fibre, however, the real image is manifest on the end or image plane ofthe fibre bundle. This means that any foreign materials such as dust, dirt, oils, and the like adhere the end of the fibre bundle and in the image plane. Such foreign materials may be visible in the image and thus distorts or interferes with the image presented directly to the eye or to a video screen.

The present invention is based upon the discovery that a window of selected thickness may be either glued to the end of an imaging fibre or located away from the fibre so the foreign material adhering to the external surface of the window is out of focus of the viewing optics.

According to the invention, a window of clear glass is disposed over the end or image plane of the imaging fibre. The free surface of the window facing the viewing optic is thus spaced from the image plane, which is the end ofthe fibre, and which is at the focus of the viewing optics. Thus, any dirt that settles on the free surface of the glass is out of focus with respect to said view optic. The window may be in contact with the end of the fibre or it may be in spaced relationship therewith. When the window is in contact with the end of the fibre, the thickness of the glass is sufficient so that the free surface is out offocus. When the widow is spaced away from the end of the fibre, the spacing is such that both surfaces of the window are out of focus, so that dust captured within the system are unlikely to interfere with the image.

Examples of apparatus according to the present invention will now be described with reference to the accompanying drawings, in which:

Fig. 1 is a schematic side sectional view of a protective window for an endoscope in accordance with the embodiment of the invention.

Fig. 2 is a view of the proximal end of the fibre.

Fig. 3 is a schematic side sectional relation of another embodiment of the invention; and

Fig. 4 is an exploded view of an exemplary embodiment ofa endoscope employing an arrangement in accordance with the invention.

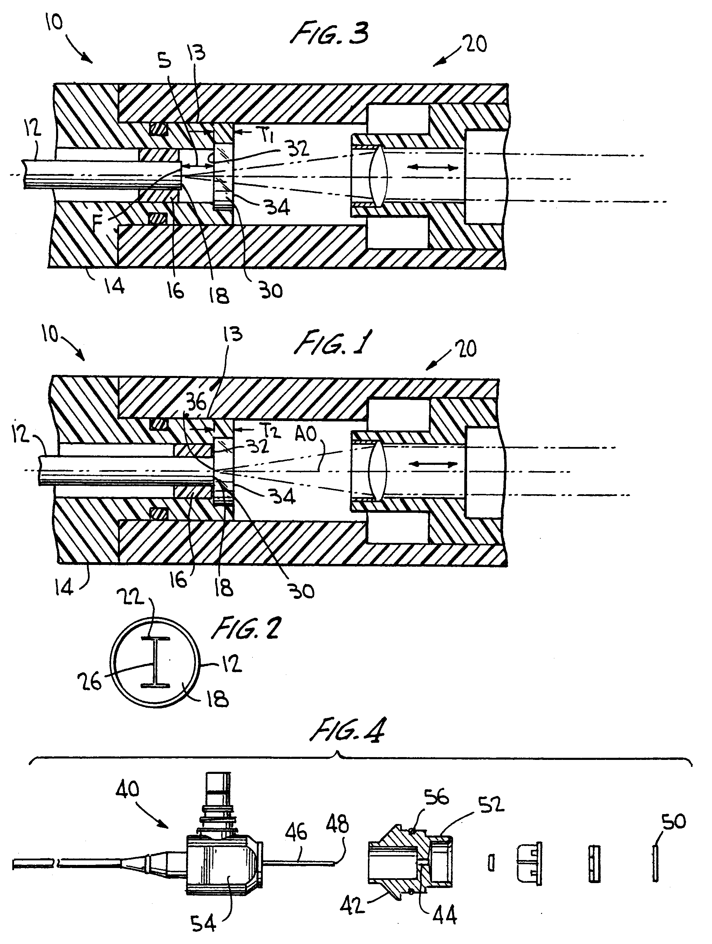

Fig. 1. is an illustration of an embodiment of the invention showing a fragmentary side sectional view of a proximal end of an endoscope 10 having an optical axis AO. It should be understood that other components not shown in the schematic illustration Fig. 1 may be present, but it is not necessary to show the same in order to fully understand the invention. In the arrangement of Fig. 1, imaging fibre 12 is secured within the proximal end 13 of an endoscope housing 14 by means of a bushing 16 or the like. The proximal end 18 of the fibre forms the image plane, that is, the image is formed in the plane format of said proximal end 18. Viewing optics 20 (shown schematically as a lens holder and which, as well known to those skilled in the art, may be an eye piece for a human observer as shown or a suitable viewing means for a television camera or the like) is located on the optical axis AO and is focused on the proximal end 18 of the fibre 12 as shown. In Fig. 2, the viewing optic 20 views a viewable image 22 (represented by the letter I), on the proximal end 18 of the fibre. As is shown in Fig. 2, if dirt or debris 26 adheres to the proximal end 18 of the fibre 12, the dirt 26 shows up on the image 22 as an artifact.

An optically clean protective window 30 is secured in the proximal end 13 ofthe housing 14 as shown. The window 30 may be any appropriate optical material such as fused silica or the like. The window 30 has respective interior and exterior or proximal and remote surfaces 32 and 34. In the embodiment illustrated in Fig. 1, the proximal or interior surface 32 is fixed to the proximal end 18 of the fibre by means of, for example, an index matching adhesive 36. The fibre 12 and the viewing optics 20 are arranged such that the focus F ofthe viewing optics 20 is in the formal plane on the proximal end 18 of the fibre 12. In accordance with such an arrangement, the image plane of the fibre is protected and sealed to the environment. Under such circumstances, the dirt or debris 26 on the remote or exterior surface 34 of the window 30 is out of the focal plane 18 of the imaging fibre 12 and is out of focus F of the viewing means 20. Accordingly, the dirt or debris 26 does not interfere with or show up in the image.

In another embodiment of the invention illustrated in Fig. 3, where similar reference numerals are used, the proximal end 18 of the fibre 12 is in spaced relationship by spacing S with the interior surface 32 ofthe protective window 30. In the arrangement illustrated in Fig. 3, both surfaces 32 and 34 of the protective window 30 are out ofthe focus F of the viewing optics 20. Thus, any defects or debris 26 on such surfaces are unlikely to show up in the image 22. In the arrangement ofFig. 3, it is unnecessary to adhere the proximal end of the fibre to the window. In the arrangement of Fig. 1, the thickness T1 of the glass window 30 is sufficient to move the external surface 34 well away from the focal plane of the fibre and out of focus F. In Fig. 3 the thickness T2, of the window 30, may be reduced and the spacing s is such that both surfaces 32 and 34 are well out of the focus F of the viewing optics 20.

Fig. 4 illustrates an exemplary embodiment, in exploded form, ofan endoscope 40 having a bushing 42 with a central opening 44 for receiving a fibre optic 46 therein. The proximal end 48, of the fibre 46, is secured in the opening 44 of the bushing 42. A protective window 50, in accordance with the invention may be secured in spaced relationship with the proximal end 48 of the fibre 46 by means of annular wall portions 52 which extend forwardly from the bushing 42 as shown. The bushing 42 is sealed within the body 54 ofthe endoscope 40 by means of O-rings 56. The fibre optic 46 is thus sealed within the endoscope 40 and is less likely to become contaminated by debris and the region.

## Claims

1. A protective window for an endoscopic device having a fibre optic with an image plane formed on an end of the fibre optic, said image plane being positioned in use in optical relationship at the focus of a viewing device of the protective window, the protective window comprising:
positioning means for positioning the end of the fibre optic on an axis of the positioning means; and
a transparent window secured to the positioning means on the axis, adjacent the end of the fibre optic, for protecting the end of the fibre optic from contamination by particulate matter, the transparent window having surfaces respectively proximal and remote from the end of the fibre optic, the remote surface being spaced from the end of the fibre optic out of the focus of the viewing device.

2. The protective window of claim 1, wherein the proximate surface of the window is in spaced relation with the end of the fibre optic such that both surfaces are out of the focus of the viewing device.

3. The protective window of claim 1, wherein the proximate surface of the window is secured and in intimate contact with the end of the fibre optic.

4. The protective window of claim 3, wherein an index matching adhesive secures the proximal end of the fibre optic to the proximate surface of the window.

5. The protective window of claim 1, wherein the positioning means comprises a bushing having a central axial opening for receiving the fibre optic therein and outer wall portions for engaging the endoscope.

6. The protective window of claim 5, wherein the outer wall portions include an O-ring seal for hermetically sealing the bushing with respect to the endoscope.

7. The protective window of any of claims 1 to 6, wherein the window comprises fused silica.

8. A protective window according to any of claims 1 to 7, further comprising a viewing device having a focal plane co-planar with the image plane of the fibre optic.

9. An endoscope having a protective window according to any of claims 1 to 8.
